# EUROPEAN PATENT APPLICATION

(11) **EP 2 332 563 A2**
(43) Date of publication of application: **15.06.2011**
(21) Application number: 10192504.8
(22) Date of filing: 13.10.2005
(51) Int. Cl.: A61K 38/17, A61P 7/00, A61P 29/00, A61P 31/04, A61P 43/00

(54) **Use of BAFF to treat sepsis**

(30) Priority: 13.10.2004 US 618438 P
(62) Divisional of application: 05810864.8
(71) Applicant: The Washington University, St. Louis, MO 63130 (US)
(72) Inventor: Hotchkiss, Richard S., Chesterfield, MO 63017 (US)
(74) Representative: Pohlman, Sandra M.

(57) **Abstract**

The disclosure provides compositions and methods for treating sepsis. The methods include administering to a mammal (e.g., a human) in need of such treatment, a pharmaceutical composition containing soluble BAFF in amount sufficient to treat sepsis, severe sepsis, and/or septic shock.

## Description

### Field of the Invention

This invention relates to treatment of sepsis. The invention further relates to uses of pharmaceutical compositions comprising BAFF (B Cell Activation Factor from the TNF family).

### Background of the Invention

Sepsis is a spectrum of clinical conditions caused by the immune response of a host to infection or trauma and characterized by systemic inflammation and coagulation (Mesters et al. (1996) Thromb. Haemost., 75:902-907; Wheeler et al. (1999) N. Engl. J. Med., 340:207-214). It ranges from a systemic inflammatory response to organ dysfunction, to multiple organ failure leading to death. At an advanced stage, the disease progresses to severe sepsis, the condition characterized by at least one acute organ dysfunction. Ultimately, severe sepsis leads to the final stage, septic shock, which is characterized by hypoperfusion and hypotension that is unresponsive to fluid resuscitation.

Sepsis is the leading cause of death in critically ill patients. An estimated 750,000 people per year develop severe sepsis in the United States alone, more than 210,000 of whom die (Angus et al. (2001) Crit. Care Med., 29:1303).

Currently, the mainstay of sepsis treatment is antimicrobial chemotherapy, removal of the source of infection, and hemodynamic, respiratory, and metabolic support. (For a review of sepsis pathophysiology and current treatment strategies, see, e.g., Hotchkiss et al. (2003) N. Engl. J. Med., 348(2):138-150.) Newer treatments under investigation include the use of anti-endotoxin agents and anti-cytokine agents. These treatments are based on the concept that sepsis represents an uncontrolled inflammatory response. However, numerous agents that interfere directly or indirectly with the actions of inflammatory mediators (e.g., corticosteroids, anti-endotoxin antibodies, tumor necrosis factor (TNF) antagonists, interleukin(IL)-1 receptor antagonists) have not been effective in preventing the death of patients with severe sepsis or septic shock (Munford (1998) Harrison's Prin. Int. Med., 14th ed., 1:776).

The failure of the anti-inflammatory agents has led investigators to question whether death in sepsis patients indeed results from uncontrolled inflammation (see, e.g., Hotchkiss-et-al. (2003) N. Engl. J. Med., 348:138; Warren (1997) N. Engl. J. Med., 336:952; Zeni et al. (1997) Crit. Care Med., 25:1095; Natanson et al. (1994) Ann. Intern. Med., 120:771; Nelson (1999) Am. J. Respir. Crit. Care Med., 159:1365). One reason for failure of anti-inflammatory strategies in sepsis may be a change in the syndrome over time. At the initial stage, sepsis involves increases in inflammatory mediators (e.g., TNF-α, interferon (INF) γ, IL-2). As sepsis progresses, there is a shift toward an anti-inflammatory, immunosuppressive state characterized by the presence of anti-inflammatory cytokines (e.g., IL-4 and IL-10) (see Hotchkiss et al. (2003) N. Engl. J. Med., 348:138). Clinical features of advanced sepsis are consistent with immune suppression, such as loss of delayed hypersensitivity, an inability to clear infection, and a predisposition to nosocomial infections (Meakins et al. (1977) Ann. Surg., 186:241; Lederer et al. (1999) Shock, 11:153; Oberholzer et al. (2001) Shock, 16:83). In more prolonged sepsis, a profound loss of B cells and CD4 T cells has been observed (Hotchkiss et al. (2001) J. Immunol., 166:6952-6963).

Therefore, there exists a need in the art to develop new therapeutic strategies and agents to treat or prevent sepsis, particularly, its more severe, prolonged forms.

### SUMMARY OF THE INVENTION

The present invention provides compositions and methods for treating sepsis. The methods include administering to a mammal, e.g., a human, having sepsis or at risk for developing sepsis, a soluble BAFF, e.g., a pharmaceutical composition containing soluble BAFF, in amount sufficient to treat sepsis, severe sepsis, and/or septic shock.

As used herein, "soluble BAFF" is a polypeptide that comprises a sequence at least 85% identical (preferably, at least 90%, 95%, 98%, 99%, or 100% identical) to the TNF-like domain of BAFF and lacks a transmembrane domain. In certain embodiments, the soluble BAFF is a polypeptide comprising all or a substantial part (e.g., at least 85%, 90%, 95% or greater) of the TNF-like domain of BAFF.

In the methods of the inventions, soluble BAFF is administered in one or more of the following periods: (1) prior to the onset of sepsis; (2) during initial sepsis but before the onset of severe sepsis; (3) during severe sepsis but before the onset of septic shock; (4) after the onset of septic shock. The methods of the invention are useful in sepsis patients with hypoimmune status, particularly, in patients who exhibit a significant loss of B cells and/or CD4 T cells (e.g., those with absolute counts of circulating lymphocytes below 700 per mm³). The invention can also be of particular use in sepsis patients with prolonged sepsis and other forms of sepsis as discussed below. In some embodiments, soluble BAFF can be administered for a period of time and/or in an amount sufficient to reduce (e.g., to substantially reduce) one or more of: sepsis-related apoptosis of lymphocytes, organ dysfunction, and inflammatory response.

As used herein, the terms "to treat," "treating," and "treatment" refer to administering a therapy in an amount, manner, and/or mode effective to improve or ameliorate a symptom or parameter that characterizes a pathological condition; to reduce the severity of a symptom or parameter that characterizes a pathological condition; to prevent, slow or reverse progression of the pathological condition; or to prevent one or more symptom or parameter of the pathological condition.

The foregoing summary and the following description are not restrictive of the invention as claimed.

### BRIEF DESCRIPTION OF THE FIGURES

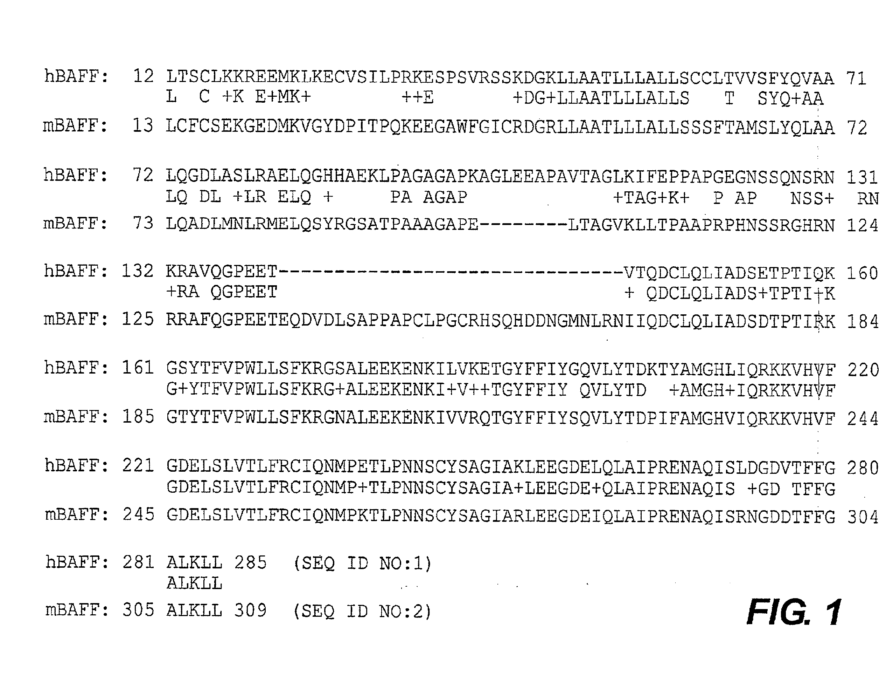

**Figure 1** shows an alignment of human (amino acids 12-285) and mouse (amino acids 13-309) BAFF sequences. "+" denotes a conservative change.

**Figure 2** shows a survival curve for mice that were administered either saline or 3 mg/kg BAFF twice daily for 3 days before and on the day of sepsis induction.

### BRIEF DESCRIPTION OF THE SEQUENCES

**SEQ ID NO:1** and **SEQ ID NO:2** represent full-length amino acid sequences of human and mouse BAFF, respectively.

**SEQ ID NO:3** is an illustrative generic sequence of soluble BAFF based on the alignment of human and mouse BAFF sequences.

### DETAILED DESCRIPTION OF THE INVENTION

The invention provides methods of treating a mammal having, or at risk of having, sepsis, which include administering a composition containing soluble BAFF in an amount and/or for a period of time sufficient to treat sepsis, severe sepsis, and/or septic shock. In the experiments described herein, BAFF (in particular, a soluble polypeptide comprising the TNF-like domain of human BAFF) was administered to mice prior to the induction of sepsis. Following the induction of sepsis, mice treated with BAFF exhibited significantly higher survival rates compared to the control mice that did not receive BAFF. Further, treatment with BAFF reduced sepsis-related apoptosis of lymphocytes.

### BAFF compositions

BAFF (also known as BLyS, TALL-1, THANK, zTNF4, neutrokine α, NTN2, TNSF13b, Kay ligand, and MARCH) is one of the recently discovered members of the TNF family. Briefly, BAFF has been implicated in costimulation of B cells (Moore et al. (1999) Science, 285:260-263; Schneider et al. (1999) J. Exp. Med., 189:1747-1756; Mukhopadhyay et al. (1999) J. Biol. Chem., 274:15978-15981); increased B cell proliferation (Moore et al. (1999) Science, 285:260-263); and increased survival of normally deleted B cells (Khare et al. (2000) Proc. Natl. Acad. Sci. USA, 97:3370-3375; Gross et al. (2000) Nature, 404:995-999; Mackay et al. (1999) J. Exp. Med., 190:1697-1710). For a review on BAFF, see, e.g., Mackay et al. (2002) Nature Reviews: Immunology, 2:465-475; and Kalled et al. (2003) Expert Opin. Ther. Targets, 7(1):115-123 and OMIM entry No. 603969.

The amino acid and nucleic acid sequences of naturally occurring full-length human BAFF are available under GenBank™ accession Nos. Q9Y275 (SEQ ID NO:1) and AF136293. The amino acid and nucleic acid sequences of full-length mouse BAFF are available under GenBank™ accession Nos. Q9WU72 (SEQ ID NO:2) and AF119383. Full-length BAFF is a type II membrane protein having intracellular, transmembrane, and extracellular domains. In human BAFF, these domains are comprised approximately (e.g., ±2 or 3 residues) of amino acids 1-46, 47-73, and 74-285 of SEQ ID NO:1, respectively. In mouse BAFF, these domains are comprised approximately (e.g., ±2 or 3 residues) of amino acids 1-53, 53-73, 74-309 of SEQ ID NO:2, respectively. A naturally occurring soluble form of BAFF exists, in which proteolytic cleavage occurs between amino acids R133 and A134 in human BAFF (amino acids R125 and A126 in mouse BAFF as predicted), resulting in a water-soluble biologically active C-terminal portion of BAFF.

Compositions suitable for use in methods of invention include soluble BAFF. Such soluble forms of BAFF generally do not comprise the transmembrane and intracellular domains. Since naturally occurring soluble BAFF does not comprise a portion of the extracellular domain (i.e., amino acids 74-133 of SEQ ID NO:1 or amino acids 74-125 of SEQ ID NO:2), soluble BAFF of the invention may likewise exclude these regions.

Within the extracellular domain, BAFF shares identity with other TNF family members: 28.7% with APRIL, 16.2% with TNF-α, and 14.1 % with lymphotoxin(LT)-α. The extracellular domain of BAFF, including naturally occurring soluble BAFF, therefore contains the TNF-like domain delimited approximately (e.g., ±2 or 3 residues) by amino acids 145-284 in SEQ ID NO:1 \ and amino acids 170-305 in SEQ ID NO:2. Accordingly, in certain embodiments, the soluble BAFF is a polypeptide comprising all or a substantial part of the TNF-like domain of BAFF, e.g., amino acids 145-284 of SEQ ID NO:1 (human BAFF), amino acids 170-305 of SEQ ID NO:2 (mouse BAFF), amino acids 11-150 of SEQ ID NO:3 (generic sequence), or a sequence at least 85%, 90%, or 95% identical thereto. The TNF-like domain of human BAFF is greater than 85% identical to mouse BAFF.

Percent identity between two amino acid sequences may be determined by standard alignment algorithms such as, for example, Basic Local Alignment Tool (BLAST) described in Altschul et al. (1990) J. Mol. Biol., 215:403-410, the algorithm of Needleman et al. (1970) J. Mol. Biol., 48:444-453, or the algorithm of Meyers et al. (1988) Comput. Appl. Biosci., 4:11-17. Such algorithms are incorporated into the BLASTN, BLASTP, and "BLAST 2 Sequences" programs (see www.ncbi.nlm.nih.gov/BLAST). When utilizing such programs, the default parameters can be used. For example, for nucleotide sequences the following settings can be used for "BLAST 2 Sequences": program BLASTN, reward for match 2, penalty for mismatch -2, open gap and extension gap-penalties 5 and 2 respectively, gap x_dropoff 50, expect 10, word size 11, filter ON. For amino acid sequences the following settings can be used for "BLAST 2 Sequences": program BLASTP, matrix BLOSUM62, open gap and extension gap penalties 11 and 1 respectively, gap x_dropoff 50, expect 10, word size 3, filter ON.

In some embodiments, the method includes evaluating the subject, before and/or after treatment, for one or more of: white blood cell count, platelet count, blood culture bacteria count, blood gas, kidney function.

In nonlimiting illustrative embodiments, soluble BAFF comprises amino acids 134-285 of SEQ ID NO:1, or N- and/or C-terminal truncations thereof. For example, the N-terminus of soluble BAFF may be between residues 134-170 of SEQ ID NO:1, e.g., the N-terminus of soluble BAFF may extend up to and include amino acid 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, or 170; while independently, the C-terminus be between residues 250-285 of SEQ ID NO:1, e.g., it may extend up to and include amino acid 285, 284, 283, 282, 281, 280, 279, 278, 277, 276, 275, 274, 273, 272, 271, 270, 269, 268, 267, 266, 265, 264, 263, 262, 261, 260, 259, 258, 257, 256, 255, 254, 253, 252, 251, 250 of SEQ ID NO:1. In one embodiment, soluble BAFF comprises amino acids 136-285 of SEQ ID NO:1.

In other nonlimiting illustrative embodiments, soluble BAFF comprises amino acids 126-309 of SEQ ID NO:2, or an N- and/or C-terminal truncations thereof. For example, the N-terminus of soluble BAFF may extend up to and include amino acid 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, or 170; while independently, the C-terminus may extend to and include amino acid 309, 308, 307, 306, 305, 304, 303, 302, 301, 300, 299, 298, 297, 296, 295, 294, 293, 292, 291, 290, 289, 288, 287, 286, 285, 284, 283, 282, 281, 280, 279, 278, 277, 276, 275, 274, 273, 272, 271, or 270 of SEQ ID NO:2.

Composition comprising soluble BAFF suitable for use in the methods of the invention further include such derivatives of BAFF in which the native BAFF sequence is mutated, partially deleted, and/or contains one or more insertions so long as changes to the native sequence do not substantially affect the biological activity of the molecule. Such changes may involve, for example, conservative amino acid substitution(s) according to Table 1. Nonlimiting examples of such changes are shown in SEQ ID NO:3. It is contemplated that soluble BAFF may contain no more than, for example, 50, 40, 30, 25, 20, 15, 10, 5, 4, 3, 2, or 1 amino acids that are substituted, deleted, or inserted relative to the naturally occurring BAFF sequences as set out from amino acid (aa) 136 to aa 285 of SEQ ID NO:1 or from aa 126 to aa 309 of SEQ ID NO:2.

**Table 1**

| Original Residues | Exemplary Substitutions |
|---|---|
| **Ala (A)** | Val, Leu, Ile |
| **Arg (R)** | Lys, Gln, Asn |
| **Asn (N)** | Gln |
| **Asp (D)** | Glu |
| **Cys (C)** | Ser, Ala |
| **Gin (Q)** | Asn |
| **Gly (G)** | Pro, Ala |
| **His (H)** | Asn, Gln, Lys, Arg |
| **Ile (I)** | Leu, Val, Met, Ala, Phe, Norleucine |
| **Leu (L)** | Norleucine, Ile, Val, Met, Ala, Phe |
| **Lys (K)** | Arg, 1,4-Diamino-butyric Acid, Gln, Asn |
| **Met (M)** | Leu, Phe, Ile |
| **Phe (F)** | Leu, Val, Ile, Ala, Tyr |
| **Pro (P)** | Ala |
| **Ser (S)** | Thr, Ala, Cys |
| **Thr (T)** | Ser |
| **Trp (W)** | Tyr, Phe |
| **Tyr (Y)** | Trp, Phe, Thr, Ser |
| **Val (V)** | Ile, Met, Leu, Phe, Ala, Norleucine |

In some embodiments, soluble BAFF further comprises a heterologous amino acid sequence, e.g., a portion of one or more proteins other than BAFF, covalently bound to the BAFF portion at the latter's N- and/or C-terminus, and optionally further comprising a linker. The non-BAFF protein can be, for example, an immunoglobulin (e.g., the Fc portion of an immunoglobulin of any type or subtype (e.g., IgG (IgG₁, IgG₄), IgA, IgE, and IgM)), albumin, APRIL, or an affinity tag (e.g., myc-tag, His-tag). Soluble BAFF may also be linked to nonproteinaceous polymers, e.g., polyethylene glycol (PEG) and polypropylene glycol.

Additional soluble BAFF compositions suitable for use in the methods of the invention and methods of making such compositions are described in, e.g., in United States Patents No. 6,689,579; 6,475,986; 6,297,736; United States Patent Application No. 09/911,777; United States Patent Application Publication Nos. 2003/0175208; 2002/0064829; 2003/0022239; 2003/0095967; 2002/0037852; 2002/0055624; 2001/0010925; 2003/0023038; 2003/0119149; 2003/0211509; PCT Application Publication Nos. WO 99/117791; WO 00/43032; WO 98/27114; WO 98/18921; WO 98/55620; WO 99/12964; WO 99/11791; WO 00/39295; WO 00/26244; WO 01/96528; WO 02/15930; WO 03/033658; WO 03/022877; WO 03/040307; WO 03/050134; WO 03/035846; WO 03/060072; WO 03/060071; WO 04/016737; WO 00/43032; WO 00/47740; WO 00/45836; and EPC Application Publication No. 1146892.

The biological activity of soluble forms of BAFF may be evaluated using one or more of the following assays:
(1) B cell proliferation assay as described in, e.g., Scheider et al. (1999) J. Exp. Med., 189(11):1747-1756;
(2) B cell survival assay as described in, e.g., Batten et al. (2000) J. Exp. Med., 192(10):1453-1465;
(3) NFκB assay as described in, e.g., Claudio et al. (2002) Nature Imm., 3(10):898-899.
(4) Ig secretion assay as described in, e.g., Moore et al. (1999) Science, 285:260-263; and
(5) in vivo treatment of mice as described here or in, e.g., Moore et al. (1999) Science, 285:260-263.

The ability of BAFF to bind to one of its receptors (e.g., TACI, BCMA, BAFF-R) may optionally be used to pre-screen soluble BAFF forms before or in conjunction with evaluating their biological activity. Suitable receptor binding assays are described in, e.g., Gavin et al. (2003) J. Biol. Chem., 278(40):38220-38228. Accordingly, in some embodiments, soluble BAFF comprises a fragment of the BAFF extracellular domain capable of binding to a BAFF receptor. It is contemplated that such a fragment may comprise one or more regions of at least 20, 30, 40, 50, 60, 70, 80, 90, 100, 110, or 120 contiguous amino acids of SEQ ID NO:1, SEQ ID NO:2, SEQ ID NO:3, or soluble BAFF derived therefrom.

Methods of making soluble BAFF are well known in the art (see, e.g., references cited herein and Fernandez et al. (1999) Gene Expression-Systems, Academic Press).

### Uses and Methods of Administration

In the methods of the inventions, soluble BAFF is administered to a subject in one or more of the following periods: (1) prior to the onset of sepsis; (2) during initial sepsis but before the onset of severe sepsis; (3) during severe sepsis but before the onset of septic shock; (4) after the onset of septic shock.

The subject treated is a "mammal," e.g., human, monkey, pig, mouse, or rat.

Soluble BAFF can be administered to a mammal prior to the onset of sepsis if a mammal is at high risk of developing sepsis (e.g., the mammal has suffered multiple trauma, burn injury, extensive surgical procedures). For example, soluble BAFF may be administered up to three weeks before surgery, catheterization, mechanical ventilation, or other invasive procedure associated with a risk of causing sepsis.

Soluble BAFF can be administered during initial sepsis but before the onset of severe sepsis, i.e., during the early stage of sepsis. The early stage of sepsis corresponds with an inflammatory response, characterized by increased levels of white blood cells (e.g., 30%, 50%, 70%, 100%, 150%, 200%, 300% above the upper end (i.e., 11,000 per mm³) of the normal range); and/or increased levels of pro-inflammatory cytokines (TNF-α, INF γ, and IL-2 e.g., more than 5, 10, 50, 100, or 500-fold above detectable levels).

Soluble BAFF can be administered during severe sepsis but before the onset of septic shock. Alternatively, soluble BAFF may be administered after the onset septic shock. These advanced stages of sepsis correspond with an immunosuppressive state. Accordingly, the methods of the invention can be practiced in subjects with hypoimmune status, e.g., those who exhibit a significant loss of B cells and/or CD4 T cells (e.g., absolute counts of circulating lymphocytes below the lower end (i.e., 700 per mm³) of the normal range by 10%, 20%, 30%, 40%, 50%, 60%, 70%, such as below 630, 600, 550, 500, or 450 per mm³; low absolute counts of total B cells (e.g., below 100, 70, 50, 30, 15, 10 per mm³; and/or low absolute counts of CD8 T cells (e.g., below 500, 450, 400, 350, 300, 250, 200 per mm³). Subjects with hypoimmune status may also exhibit high levels of anti-inflammatory cytokines (e.g., IL-4, IL-10, e.g., more than 5; 10, 50, 100 or 500-fold above detectable levels), and/or loss of delayed hypersensitivity.

Furthermore, the invention can also be used to treat prolonged sepsis (e.g., 3, 4, 5, 6, 7, 8, 9, 10 days or longer), and other forms of sepsis as provided below. In some embodiments, the invention provides a method of reducing apoptosis of lymphocytes in a subject having prolonged sepsis. In such embodiments, the method comprises administering soluble BAFF to the subject in an amount and for a period of time sufficient to reduce apoptosis of lymphocytes (e.g., B cells, CD8 T cells) by at least 10%, 20%, 30%, 40%, 50%, 60%, or 70%. The methods for assessing apoptosis of lymphocytes are known and described here.

Sepsis includes, but is not limited to, conditions such as bacteremia, septicemia, periotonitis, systemic inflammatory response syndrome (SIRS), refractory septic shock, and multiple-organ dysfunction syndrome (MODS).

The invention can be used in sepsis patients whose predicted mortality rate on the Acute Physiology and Chronic Health Evaluation (APACHE) II scale is at least 20%, 25%, 30%, 35%, 40%, 50%, 60%, 70%, 80%, or more. (For the description of the APACHE II scale, see, e.g., Knaus et al. (1985) Crit. Care Med., 13:818-829.)

"Administration" is not limited to any particular formulation, delivery system, or route and may include, for example, parenteral (including subcutaneous, intravenous, intramedullary, intraarticular, intramuscular, or intraperitoneal injection) rectal, topical, transdermal, or oral (for example, in capsules, suspensions, or tablets). Administration may be provided in a single dose or repeatedly, and in any of a variety of pharmaceutical compositions containing physiologically acceptable salt forms, and/or with an acceptable pharmaceutical excipients. Physiologically acceptable salt forms and pharmaceutical formulations and excipients are known (see, e.g., 2004 Physicians' Desk Reference® (PDR) (2003) Thomson Healthcare, 58th ed; Gennado et al., (2000), 20th ed, Lippincott, Williams & Wilkins) Remington: The Science and Practice of Pharmacy.

Antimicrobial agents that may be administered in conjunction with soluble BAFF include, without limitation, aminoglycosides (e.g., amikacin, gentamicin, kanamycin, tobramycin, neomycin, netilmicin, and streptomycin), cephalosporins (such as cefadroxil, cefazolin, cephalexin, cephalothin, cefaclor, cefoxitin, cefuroxime, loracarbef, ceftazidime, and cefepime), macrolides (e.g., clarithromycin, erythromycin, and azithromycin), penicillins (e.g., amoxicillin, oxacillin, piperacillin, ticarcillin, and ampicillin), quinolones (e.g., ciprofloxacin, gemifloxicin, ofloxicin, levofloxicin, norfloxicin, enoxacin, trovofloxacin, and alatrofloxacin), sulfonamides, tetracyclines, and other antimicrobials (e.g., aztreonam, clindamycin, vancomycin, iodoquinol, and linezolid). Other antimicrobials include, without limitation, antifungals, antimalarials, antiparasitics, antituberculars, and antivirals.

Various other adjunctive agents may also be used in combination with soluble BAFF to treat sepsis. Examples of such adjunctive agents include sympathomimetic amines (e.g., norepinephrine, epinephrine, isoproterenol, dopamine, and dobutamine), anti-inflammatory agents (e.g., methylprednisolone, indomethacin, phenylbutazone, betamethasone, hydrocortisone, and dexamethasone), anticoagulants (e.g., heparin, anti-thrombin III, and coumarin-type drugs), diuretics (e.g., furosemide and ethacrynic acid), and opiate antagonists. Other agents that may be used in combination with soluble BAFF further include, without limitation, activated protein C, insulin, cytokines, cytokine antagonists, and therapies aimed at optimizing cardiac preload, afterload, and contractility, A treating physician will determine the exact dosages and regimens for soluble BAFF and adjunct therapeutics. As a starting point, a therapeutically effective dose of soluble BAFF may be in the range of 0.005 to 5 milligram per kilogram body weight. For example, the dose may be administered one time or a plurality of times, e.g., 0.05 to 0.5 mg/kg/day.

Therapeutically effective dosages achieved in one animal model may be converted for use in another animal, including humans, using known conversion factors (see, e.g., Freireich et al. (1966) Cancer Chemother. Reports, 50(4):219-244 and Table 2 for equivalent surface area dosage factors).

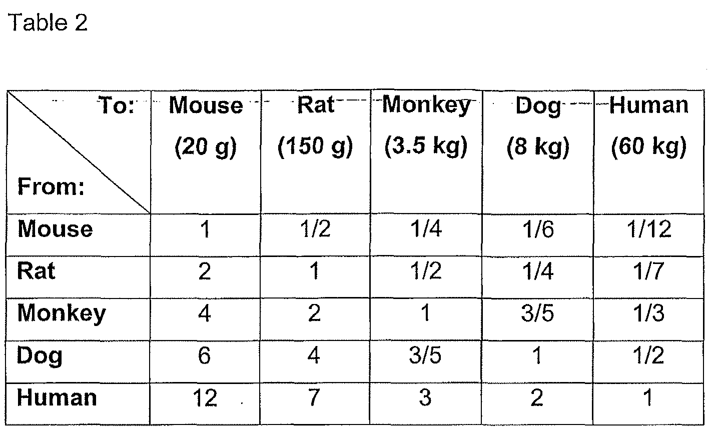

The following Examples provide illustrative embodiments. The Examples do not in any way limit the invention. One of ordinary skill in the art will recognize numerous modifications and variations that may be performed within the scope of the present invention. Such modifications and variations are therefore encompassed by the invention.

### EXAMPLES

### Example 1: BAFF Improves Survival in Sepsis

Mice (6-8 week old male C57BL6) were anesthetized, the peritoneum was opened sterilely, and the cecum was isolated and ligated with a 4-0 silk suture and punctured twice with a 25-gauge syringe needle. Sham-operated mice received cecal manipulation only. The abdominal cavity was closed in two layers. One milliliter of 0.9% saline was injected subcutaneously to compensate for third space fluid losses. Mice were allowed free access to food and water throughout the study. Mortality typically began at 24 hours and continued for the following 2-5 days. Survival was recorded at 7 days since mice had usually made a complete recovery from sepsis at this point.

The mouse cecal ligation and puncture (CLP) model of sepsis is a clinically relevant model of sepsis (peritonitis) that has been validated in many laboratories (See, e.g., Baker et al. (1983) Surgery, 94:331; Baker et al. (1991) Arch. Surg., 126:253; Remick et al. (2000) Shock, 13:110). Positive blood cultures in all CLP mice but none in sham mice (See, e.g., Hotchkiss et al. (2000) Nat. Immunol., 1:496) were used to confirmed the presence of sepsis. The bacteria, such as *E. coli, Enterobacter cloacae, Streptococcus faecium,* and *Enterobacter faecium,* detected in the blood of CLP mice are also known to be common pathogens in human sepsis.

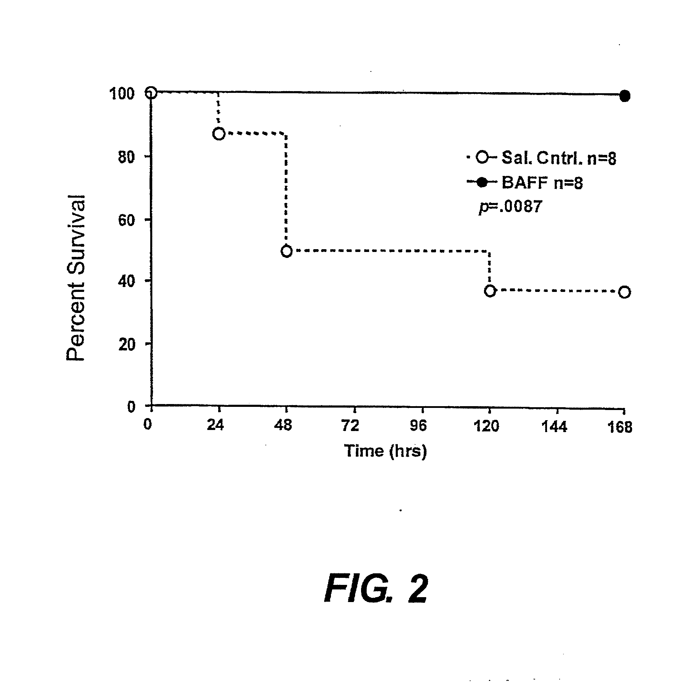

To confirm that soluble BAFF administered before the onset of CLP would improve survival in sepsis, mice were treated with 3 mg/kg of soluble BAFF (amino acids 136-285 or human BAFF; see, e.g., Thompson et al. (2001) Science, 293:2108-211, Suppl.) or saline as control. The injections with BAFF or saline were administered twice daily for 3 days before CLP. Mice also received a dose of BAFF or saline on the day of sepsis surgery. As shown in Figure 2, all mice treated with BAFF survived sepsis. In contrast, only 37.5% of the mice treated with saline were alive 7 days after the induction of sepsis. Therefore, soluble BAFF dramatically improves survival in sepsis.

### Example 2: BAFF Reduces Sepsis-Induced Lymphocyte Apoptosis

To confirm that BAFF administered before the onset of sepsis decreases sepsis-induced lymphocyte apoptosis, 5 C57BL6 mice were treated with BAFF via subcutaneous injection at a dose of 3 mg/kg twice daily for 5 days before CLP. Five control mice received saline only. Blood was collected from mice approximately 6 hours after the surgery.

Apoptosis of circulating lymphocytes was evaluated using flow cytometry and annexin V/7AAD labeling kit (BioSource, No. PHN1018). As shown in Table 3, the pretreatment with BAFF significantly decreased lymphocyte apoptosis. The mean value for lymphocyte apoptosis in the saline-treated control group was 36.09±4.76%. In contrast, the mean value for lymphocyte apoptosis in the BAFF-treated group was 20.86±3.97%. Therefore, a pretreatment with BAFF reduces sepsis-induced lymphocyte death. The reduction of lymphocyte apoptosis in advanced stages of sepsis helps decrease immune suppression thereby improving survival.

**Table 3**

| **No.** | **Percentage of apoptotic cells** | |
|---|---|---|
| | **BAFF-treated** | **Control** |
| 1 | 16.96 | 42.32 |
| 2 | 12.72 | 32.36 |
| 3 | 23.14 | 34.41 |
| 4 | 18.09 | 23.3 |
| 5 | 33.4 | 48.08 |
| **Mean±SEM** | **20.86±3.97** | **36.09±4.76** |

The specification is most thoroughly understood in light of the teachings of the references cited within the specification. The embodiments within the specification provide an illustration of embodiments of the invention and should not be construed to limit the scope of the invention. The skilled artisan readily recognizes that many other embodiments are encompassed by the invention. All publications, patents, and biological sequences cited in this disclosure are incorporated by reference in their entirety. To the extent the material incorporated by reference contradicts or is inconsistent with the present specification, the present specification will supersede any such material. The citation of any references herein is not an admission that such references are prior art to the present invention.

Unless otherwise indicated, all numbers expressing quantities of ingredients, cell culture, treatment conditions, and so forth used in the specification, including claims, are to be understood as being modified in all instances by the term "about." Accordingly, unless otherwise indicated to the contrary, the numerical parameters are approximations and may vary depending upon the desired properties sought to be obtained by the present invention. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein.

In view of the foregoing, it will be appreciated that the invention described herein *inter alia* relates to the following items:
1. A method of treating sepsis in a mammal having or at risk for developing sepsis, the method comprising administering to the mammal a soluble BAFF (B cell Activation Factor from the TNF family) in amount sufficient to treat sepsis, severe sepsis, or septic shock.
2. The method of item 1, wherein the mammal is human.
3. The method of item 1, wherein the soluble BAFF is administered to the mammal in one or more of the following periods:
   (a) prior to the onset of sepsis;
   (b) during initial sepsis but before the onset of severe sepsis;
   (c) during severe sepsis but before the onset of septic shock; and
   (d) after the onset septic shock.
4. The method of item 1, wherein the mammal has prolonged sepsis.
5. The method of item 1, wherein the mammal has predicted mortality rate of at least 20% on the APACHE (Acute Physiology and Chronic Health Evaluation) II scale.
6. The method of item 5, wherein the mammal is a human who exhibits one or more of the following:
   (a) absolute counts of white blood cells at least 30% below 11,000 per mm³;
      and
   (b) increased levels of pro-inflammatory cytokines.
7. The method of item 5, wherein the mammal exhibits significant loss of B and/or CD4 T cells.
8. The method of item 5, wherein the mammal is a human who exhibits one or more of the following:
   (a) absolute counts of circulating lymphocytes below 630 per mm³;
   (b) absolute counts of total B cells below 100 per mm³;
   (c) absolute counts of total CD8 T cells below 500 per mm³;
   (d) high level of anti-inflammatory cytokines; and
   (e) loss of delayed hypersensitivity.
9. The method of item 1, wherein the soluble BAFF comprises:
   (a) a fragment of BAFF extracellular domain capable of binding to a BAFF receptor; or
   (b) a substantial part of the TNF-like domain of BAFF;
      and does not comprise the BAFF intracellular or transmembrane domains.
10. The method of item 1, wherein soluble BAFF comprises amino acids 145-284 of SEQ ID NO:1.
11. The method of item 1, wherein the soluble BAFF comprises amino acids 169-308 of SEQ ID NO:2.
12. The method of item 1, wherein the soluble BAFF comprises amino acids 11-150 of SEQ ID NO:3.
13. The method of item 1, wherein the soluble BAFF comprises an Fc portion of an immunoglobulin.
14. The method of item 2, wherein the human has suffered multiple trauma, burn injury, or a surgical procedure.
15. The method of item 1, wherein the soluble BAFF is administered in combination with a second therapeutic agent for the treatment of sepsis.
16. The method of item 1, wherein the soluble BAFF is administered at a dosage between 0.005 and 5 mg/kg.
17. The method of item 1, wherein the soluble BAFF comprises a polypeptide at least 90% identical to amino acids 145-284 of SEQ ID NO:1.
18. The method of item 1, further comprising evaluating the human for one or more of: white blood cell count, platelet count, blood culture bacteria count, blood gas, and kidney function.
19. A method of reducing apoptosis of lymphocytes in a subject having prolonged sepsis, the method comprising administering soluble BAFF to the subject in an amount and for a period of time sufficient to reduce apoptosis of lymphocytes.
20. Use of soluble BAFF to treat or prevent sepsis, severe sepsis, or septic shock.
21. Use of soluble BAFF in the manufacture of a medicament to treat or prevent sepsis, severe sepsis, or septic shock.
22. Use of items 20 or 21, wherein sepsis, severe sepsis, or septic shock is a clinical condition further defined as septicemia, periotonitis, systemic inflammatory response syndrome (SIRS), refractory septic shock, or multiple-organ dysfunction syndrome (MODS).
23. Use according to item 20, 21, or 22, wherein the subject is human.
24. Use according to item 20, 21, or 22, wherein the soluble BAFF comprises:
   (a) amino acids 145-284 of SEQ ID NO:1;
   (b) amino acids 170-308 of SEQ ID NO:2;
   (c) amino acids 11-150 of SEQ ID NO:3;
   (d) a sequence at least 70% identical to any one of (a) - (c);
   (e) a fragment of BAFF extracellular domain capable of binding to a BAFF receptor;
   (f) substantial part of the TNF-like domain of BAFF; or
   (g) any one of (a) - (e) fused to an Fc portion of an immunoglobulin.

## Claims

1. A soluble BAFF (B cell Activation Factor from the TNF family) polypeptide for use in treating, preventing, or reducing the severity of a symptom or parameter of sepsis, severe sepsis, or septic shock in a mammal.

2. The soluble BAFF polypeptide of claim 1, wherein the mammal is human.

3. The soluble BAFF polypeptide of claim 1 or claim 2, which is to be administered to the mammal in one or more of the following periods:
(a) prior to the onset of sepsis;
(b) during initial sepsis but before the onset of severe sepsis;
(c) during severe sepsis but before the onset of septic shock; and
(d) after the onset septic shock.

4. The soluble BAFF polypeptide of any one of claims 1-3, wherein the mammal is a human who exhibits one or more of the following:
(a) absolute counts of white blood cells at least 30% above 11,000 per mm³; and
(b) increased levels of pro-inflammatory cytokines.

5. The soluble BAFF polypeptide of any one of claims 1-3, wherein the mammal is a human who exhibits one or more of the following:
(a) predicted mortality rate of at least 20% on the APACHE (Acute Physiology and Chronic Health Evaluation) II scale;
(b) significant loss of B and/or CD4 T cells;
(c) absolute counts of circulating lymphocytes below 630 per mm³;
(d) absolute counts of total B cells below 100 per mm³;
(e) absolute counts of total CD8 T cells below 500 per mm³;
(f) high level of anti-inflammatory cytokines; and
(g) loss of delayed hypersensitivity.

6. The soluble BAFF polypeptide of any one of claims 1-5, wherein the soluble BAFF polypeptide comprises:
(a) a fragment of BAFF extracellular domain capable of binding to a BAFF receptor; or
(b) a substantial part of the TNF-like domain of BAFF;
and does not comprise the BAFF intracellular or transmembrane domains.

7. The soluble BAFF polypeptide of any one of claims 1-6, wherein the soluble BAFF polypeptide is selected from the group consisting of:
(a) a polypeptide comprising amino acids 145-284 of SEQ ID NO:1;
(b) a polypeptide comprising amino acids 169-308 of SEQ ID NO:2;
(c) a polypeptide comprising amino acids 11-150 of SEQ ID NO:3;
(d) a polypeptide at least 90% identical to amino acids 145-284 of SEQ ID NO:1;
(e) a sequence at least 70% identical to any one of (a) - (c);
(f) a fragment of BAFF extracellular domain capable of binding to a BAFF receptor;
(g) substantial part of the TNF-like domain of BAFF; and
(h) any one of (a) - (g) fused to an Fc portion of an immunoglobulin.

8. The soluble BAFF polypeptide of any one of claims 1-7, wherein the soluble BAFF polypeptide comprises an Fc portion of an immunoglobulin.

9. The soluble BAFF polypeptide of any one of claims 1-8, wherein the mammal is a human that has suffered one or more of multiple trauma, burn injury, or a surgical procedure.

10. The soluble BAFF polypeptide of any one of claims 1-9, wherein the soluble BAFF polypeptide is administered in combination with a second therapeutic agent for the treatment of sepsis.

11. The soluble BAFF polypeptide of any one of claims 1-10, wherein the soluble BAFF polypeptide is administered at a dosage between 0.005 and 5 mg/kg.

12. The soluble BAFF polypeptide of any one of claims 1-11, wherein the sepsis, severe sepsis, or septic shock is a clinical condition further defined as septicemia, periotonitis, systemic inflammatory response syndrome (SIRS), refractory septic shock, or multiple-organ dysfunction syndrome (MODS).

13. The soluble BAFF polypeptide of any one of claims 1-12, wherein the mammal is a human that is to be subject to one or more invasive procedures selected from surgery, catheterization, and mechanical ventilation.

14. A soluble BAFF polypeptide for use in reducing apoptosis of lymphocytes in a subject having prolonged sepsis.


SEQUENCE LISTING

<110>  The Washington University

<120>  USE OF BAFF TO TREAT SEPSIS

<130>  WSH12257PCTEPD1

<160>  3

<170>  PatentIn version 3.1

<210>  1
<211>  285
<212>  PRT
<213>  Human

<400>  1

Met Asp Asp Ser Thr Glu Arg Glu Gln Ser Arg Leu Thr Ser Cys Leu
1               5                   10                  15

Lys Lys Arg Glu Glu Met Lys Leu Lys Glu Cys Val Ser Ile Leu Pro
            20                  25                  30

Arg Lys Glu Ser Pro Ser Val Arg Ser Ser Lys Asp Gly Lys Leu Leu
        35                  40                  45

Ala Ala Thr Leu Leu Leu Ala Leu Leu Ser Cys Cys Leu Thr Val Val
    50                  55                  60

Ser Phe Tyr Gln Val Ala Ala Leu Gln Gly Asp Leu Ala Ser Leu Arg
65                  70                  75                  80

Ala Glu Leu Gln Gly His His Ala Glu Lys Leu Pro Ala Gly Ala Gly
                85                  90                  95

Ala Pro Lys Ala Gly Leu Glu Glu Ala Pro Ala Val Thr Ala Gly Leu
            100                 105                 110

Lys Ile Phe Glu Pro Pro Ala Pro Gly Glu Gly Asn Ser Ser Gln Asn
        115                 120                 125

Ser Arg Asn Lys Arg Ala Val Gln Gly Pro Glu Glu Thr Val Thr Gln
    130                 135                 140

Asp Cys Leu Gln Leu Ile Ala Asp Ser Glu Thr Pro Thr Ile Gln Lys
145                 150                 155                 160

Gly Ser Tyr Thr Phe Val Pro Trp Leu Leu Ser Phe Lys Arg Gly Ser
                165                 170                 175

Ala Leu Glu Glu Lys Glu Asn Lys Ile Leu Val Lys Glu Thr Gly Tyr
            180                 185                 190

Phe Phe Ile Tyr Gly Gln Val Leu Tyr Thr Asp Lys Thr Tyr Ala Met
        195                 200                 205

Gly His Leu Ile Gln Arg Lys Lys Val His Val Phe Gly Asp Glu Leu
    210                 215                 220

Ser Leu Val Thr Leu Phe Arg Cys Ile Gln Asn Met Pro Glu Thr Leu
225                 230                 235                 240

Pro Asn Asn Ser Cys Tyr Ser Ala Gly Ile Ala Lys Leu Glu Glu Gly
                245                 250                 255

Asp Glu Leu Gln Leu Ala Ile Pro Arg Glu Asn Ala Gln Ile Ser Leu
            260                 265                 270

Asp Gly Asp Val Thr Phe Phe Gly Ala Leu Lys Leu Leu
        275                 280                 285

<210>  2
<211>  309
<212>  PRT
<213>  mouse

<400>  2

Met Asp Glu Ser Ala Lys Thr Leu Pro Pro Pro Cys Leu Cys Phe Cys
1               5                   10                  15

Ser Glu Lys Gly Glu Asp Met Lys Val Gly Tyr Asp Pro Ile Thr Pro
            20                  25                  30

Gln Lys Glu Glu Gly Ala Trp Phe Gly Ile Cys Arg Asp Gly Arg Leu
        35                  40                  45

Leu Ala Ala Thr Leu Leu Leu Ala Leu Leu Ser Ser Ser Phe Thr Ala
    50                  55                  60

Met Ser Leu Tyr Gln Leu Ala Ala Leu Gln Ala Asp Leu Met Asn Leu
65                  70                  75                  80

Arg Met Glu Leu Gln Ser Tyr Arg Gly Ser Ala Thr Pro Ala Ala Ala
                85                  90                  95

Gly Ala Pro Glu Leu Thr Ala Gly Val Lys Leu Leu Thr Pro Ala Ala
            100                 105                 110

Pro Arg Pro His Asn Ser Ser Arg Gly His Arg Asn Arg Arg Ala Phe
        115                 120                 125

Gln Gly Pro Glu Glu Thr Glu Gln Asp Val Asp Leu Ser Ala Pro Pro
    130                 135                 140

Ala Pro Cys Leu Pro Gly Cys Arg His Ser Gln His Asp Asp Asn Gly
145                 150                 155                 160

Met Asn Leu Arg Asn Ile Ile Gln Asp Cys Leu Gln Leu Ile Ala Asp
                165                 170                 175

Ser Asp Thr Pro Thr Ile Arg Lys Gly Thr Tyr Thr Phe Val Pro Trp
            180                 185                 190

Leu Leu Ser Phe Lys Arg Gly Asn Ala Leu Glu Glu Lys Glu Asn Lys
        195                 200                 205

Ile Val Val Arg Gln Thr Gly Tyr Phe Phe Ile Tyr Ser Gln Val Leu
    210                 215                 220

Tyr Thr Asp Pro Ile Phe Ala Met Gly His Val Ile Gln Arg Lys Lys
225                 230                 235                 240

Val His Val Phe Gly Asp Glu Leu Ser Leu Val Thr Leu Phe Arg Cys
                245                 250                 255

Ile Gln Asn Met Pro Lys Thr Leu Pro Asn Asn Ser Cys Tyr Ser Ala
            260                 265                 270

Gly Ile Ala Arg Leu Glu Glu Gly Asp Glu Ile Gln Leu Ala Ile Pro
        275                 280                 285

Arg Glu Asn Ala Gln Ile Ser Arg Asn Gly Asp Asp Thr Phe Phe Gly
    290                 295                 300

Ala Leu Lys Leu Leu
305

<210>  3
<211>  151
<212>  PRT
<213>  Artificial sequence

<220>
<223>  Generic BAFF sequence

<220>
<221>  MISC_FEATURE
<222>  (7)..(7)
<223>  None or a a stretch of amino acids up to about 30 amino acid or more), e.g, mouse
BAFF amino acids E142 to N172 (EQDVDLSAPPAPCLPGCRHSQHDDNGMNLRN)

<220>
<221>  MISC_FEATURE
<222>  (8)..(8)
<223>  V or I, or another amino acid

<220>
<221>  MISC_FEATURE
<222>  (9)..(9)
<223>  T or I, or another amino acid

<220>
<221>  MISC_FEATURE
<222>  (20)..(20)
<223>  E or D, or another amino acid

<220>
<221>  MISC_FEATURE
<222>  (25)..(25)
<223>  Q or R, or another amino acid

<220>
<221>  MISC_FEATURE
<222>  (28)..(28)
<223>  S or T, or another amino acid

<220>
<221>  MISC_FEATURE
<222>  (42)..(42)
<223>  S or N, or another amino acid

<220>
<221>  MISC_FEATURE
<222>  (52)..(52)
<223>  L or V, or another amino acid

<220>
<221>  MISC_FEATURE
<222>  (54)..(54)
<223>  K or R, or another amino acid

<220>
<221>  MISC_FEATURE
<222>  (55)..(55)
<223>  E or Q, or another amino acid

<220>
<221>  MISC_FEATURE
<222>  (63)..(63)
<223>  G or S, or another amino acid


<220>
<221>  MISC_FEATURE
<222>  (70)..(70)
<223>  K or P, or another amino acid

<220>
<221>  MISC_FEATURE
<222>  (71)..(71)
<223>  T or I, or another amino acid

<220>
<221>  MISC_FEATURE
<222>  (72)..(72)
<223>  Y or F, or another amino acid

<220>
<221>  MISC_FEATURE
<222>  (77)..(77)
<223>  L or V, or another amino acid

<220>
<221>  MISC_FEATURE
<222>  (104)..(104)
<223>  E or K, or another amino acid

<220>

<221>  MISC_FEATURE
<222>  (118)..(118)
<223>  K or R, or another amino acid

<220>
<221>  MISC_FEATURE
<222>  (125)..(125)
<223>  L or I, or another amino acid

<220>
<221>  MISC_FEATURE
<222>  (138)..(138)
<223>  L or R, or another amino acid

<220>
<221>  MISC_FEATURE
<222>  (139)..(139)
<223>  D or N, or another amino acid

<220>
<221>  MISC_FEATURE
<222>  (142)..(142)
<223>  V or D, or another amino acid

<400>  3

Gln Gly Pro Glu Glu Thr Xaa Xaa Xaa Gln Asp Cys Leu Gln Leu Ile
1               5                   10                  15

Ala Asp Ser Xaa Thr Pro Thr Ile Xaa Lys Gly Xaa Tyr Thr Phe Val
            20                  25                  30

Pro Trp Leu Leu Ser Phe Lys Arg Gly Xaa Ala Leu Glu Glu Lys Glu
        35                  40                  45

Asn Lys Ile Xaa Val Xaa Xaa Thr Gly Tyr Phe Phe Ile Tyr Xaa Gln
    50                  55                  60

Val Leu Tyr Thr Asp Xaa Xaa Xaa Ala Met Gly His Xaa Ile Gln Arg
65                  70                  75                  80

Lys Lys Val His Val Phe Gly Asp Glu Leu Ser Leu Val Thr Leu Phe
                85                  90                  95

Arg Cys Ile Gln Asn Met Pro Xaa Thr Leu Pro Asn Asn Ser Cys Tyr
            100                 105                 110

Ser Ala Gly Ile Ala Xaa Leu Glu Glu Gly Asp Glu Xaa Gln Leu Ala
        115                 120                 125

Ile Pro Arg Glu Asn Ala Gln Ile Ser Xaa Xaa Gly Asp Xaa Thr Phe
    130                 135                 140

Phe Gly Ala Leu Lys Leu Leu
145                 150